# EUROPEAN PATENT SPECIFICATION

(11) **EP 3 949 833 B1**
(45) Date of publication and mention of the grant of the patent: **05.04.2023**
(21) Application number: 20917687.4
(22) Date of filing: 03.02.2020
(51) Int. Cl.: A61B 5/00, A61B 5/24, A61B 5/276

(54) **CONTACT STATE DETECTION DEVICE AND WEARABLE APPARATUS**
KONTAKTZUSTANDSDETEKTOR UND AM KÖRPER TRAGBARE VORRICHTUNG
PROCÉDÉ DE DÉTECTION D'ÉTAT DE CONTACT ET APPAREIL VESTIMENTAIRE

(43) Date of publication of application: 09.02.2022
(73) Proprietor: SHENZHEN GOODIX TECHNOLOGY CO., LTD., Shenzhen, Guangdong 518045 (CN)
(72) Inventor: LIU, Chang, Shenzhen, Guangdong 518045 (CN); DENG, Fujian, Shenzhen, Guangdong 518045 (CN)
(74) Representative: Herrero & Asociados, S.L.
(86) International application number: PCT/CN2020/074199
(87) International publication number: WO 2021/155488

(56) References cited:
- CN-A- 103 860 165
- CN-A- 104 548 343
- CN-A- 106 667 484
- CN-A- 106 999 083
- CN-A- 108 261 196
- CN-A- 109 528 193
- CN-U- 205 251 535
- CN-U- 208 270 697
- US-A1- 2010 007 413
- US-A1- 2018 067 063
- US-A1- 2019 274 568
- US-B1- 6 516 218

## Description

### TECHNICAL FIELD

Embodiments of the present application relate to the electronic field, and more particularly, to a contact state detection apparatus and a wearable device.

### BACKGROUND

As is known to all, an electrocardiogram examination has become an import item of modern medical examinations.

In medical specialized equipment for detecting an electrocardiogram, an alarm can be raised through a lead-off detection function in a case that leads (clips for hands and feet or suction balls for a chest) fall off, to remind an operator that the leads are not correctly connected to a detected object (a human body or others), that is, the leads fall off.

With the progress of technology, especially the development of semiconductor technology, small electrocardio detection products have been widely applied to the fields of various portable and even wearable products. Although wearable devices that can be configured for electrocardio detection cannot replace specialized equipment in hospitals, they play an irreplaceable role in the fields of health surveillance and disease prevention.

However, in consideration of volume and aesthetic factors, a dry electrode with a relatively small area is used for a wearable product (normal saline is applied to a detected object when medical specialized equipment is used, to increase conductivity of the detected object), which causes that contact impedance between the dry electrode and the detected object is extremely high, or serious impedance mismatches occur, for example, occasions where the skin surface is very dry. In those scenarios, if a lead-off detection solution of traditional medical specialized equipment is adopted to perform electrode contact state detection of the wearable device, it loses effect or has some adverse effects. For example, the extremely high contact impedance or polarized voltage may cause false alarms. For another example, the extremely high contact impedance may amplify flicker noise in a detection signal, and reduce a signal-to-noise ratio that affects an electrocardio signal accordingly. Moreover, the extremely contact impedance may saturate an amplifier so that electrocardio detection cannot be effectively performed. In addition, in the lead-off detection solution of the medical specialized equipment relates to too many devices, and manufacturing costs are too high accordingly. Therefore, it is generally not able to be applied to wearable devices for electrode contact state detection.

Therefore, there is an urgent need in the art for a contact state detection solution that could reduce manufacturing costs on the basis of solving a problem of losing effect for electrode contact state detection due to high contact impedance or polarized voltage.
Document US20180067063A1 discloses a synchronous detection circuit for extracting magnitude and phase information from a waveform. The synchronous detection circuit includes a driver circuit, an analog-to-digital converter (ADC) and a controller. The driver circuit is configured to supply an input waveform at an input frequency to a load. The ADC is coupled to receive an output waveform from the load. The controller is configured for setting an oversampling rate (OSR) of the ADC. The controller is further configured to use the digital samples generated by the ADC to extract magnitude and phase information from the output waveform.
Document US20190274568A1 relates to integrated channel integrity detection and to reconstruction of electrophysiological signals. An example system includes a plurality of input channels configured to receive respective electrical signals from a set of electrodes. An amplifier stage includes a plurality of differential amplifiers, each of the differential amplifiers being configured to provide an amplifier output signal based on a difference between a respective pair of the electrical signals. Channel detection logic is configured to provide channel data indicating an acceptability of each of the plurality of input channels based on an analysis of a common mode rejection of the amplifier output signals.
Document US20100007413A1 discloses a system and method for generating output signals indicative of contact quality of a plurality of electrodes coupled to a patient. A signal generator coupled to a reference electrode injects an alternating signal into the patient. A plurality of differential amplifiers, each coupled to a respective one of the plurality of electrodes to detect an input signal from the patient, are operable to output a respective output signal in response to a respective input signal. The output signal generated by the respective differential amplifier is indicative of contact quality for the respective electrode.
Document CN208270697U discloses a wearable ECG detection device based on DC and AC detection, which comprises a DC lead shedding detection unit, a switch lead shedding detection unit, a signal acquisition unit and a processor unit. The DC lead shedding detection unit and the signal input end of the exchange lead shedding detection unit are respectively arranged through the measuring electrode and the human body contact. The signal output end of the DC lead shedding detection unit and the switching lead shedding detection unit are respectively connected with the signal output end of the signal acquisition unit and the signal input end of the processor unit. The signal output end of the signal acquisition unit is connected with the signal input end of the processor unit. By combining the advantages of direct and AC lead shedding detection modes, the workflow is optimized to obtain the best standby power consumption and signal detection quality.

### SUMMARY

A contact state detection apparatus and a wearable device are provided, which could reduce manufacturing costs on the basis of solving a problem of losing effect for electrode contact state detection due to high contact impedance or polarized voltage.

In a first aspect, a contact state detection apparatus is provided, and the contact state detection apparatus is applicable to a wearable device, and includes:
a first electrode and a second electrode, both of the first electrode and the second electrode being configured to receive an alternating current signal and a first signal to output a differential signal, the differential signal including the first signal and a second signal, the first signal being a detection signal of physiological information of a detected object, and the second signal being a signal formed after the alternating current signal is modulated by the first electrode and the second electrode; and
a sampling circuit, the sampling circuit being connected to the first electrode and the second electrode, the sampling circuit being configured to sample the differential signal to obtain a target sampling signal, and the target sampling signal including a first sampling signal corresponding to a first signal and a second sampling signal corresponding to the second signal;
where the first sampling signal is used to indicate a physiological signal of the detected object, and the second sampling signal is used to detect a contact state of the first electrode with the detected object and/or a contact state of the second electrode with the detected object.

Since a voltage difference between the first electrode and the second electrode changes when the first electrode or second electrode is in contact with the detected object (the first signal is present), differential signals output by the first electrode and the second electrode change accordingly. Equivalently, in a case that the first electrode and/or the second electrode is in contact with the detected object, a modulating signal formed after the alternating current signal is modulated by the first electrode and the second electrode changes with respect to a case that the first electrode and/or the second electrode is not in contact with the detected object. In this case, detection of physiological parameter information of the detected object could be implemented through the first sampling signal in the target sampling signal. On this basis, whether the first electrode or the second electrode and the detected object are in a contact state could be presented through the second sampling signal, to implement electrode contact state detection as well as physiological information detection at the same time.

In addition, by driving the first electrode and the second electrode by the alternating current signal to detect the physiological information of the detected object, the alternating current signal in the target sampling signal that is modulated by the first electrode and the second electrode can be multiplexed into a signal used to indicate whether the first electrode and/or the second electrode and the detected object fall off while the physiological information detection is implemented, which could reduce devices in the contact state detection apparatus, simplify the apparatus and reduce the manufacturing costs accordingly.

In some possible implementation manners, the alternating current signal is a high-frequency signal.

Since the detection signal is also a low-frequency signal, the alternating current signal is constructed as a high-frequency signal. On one hand, the first sampling signal and the second sampling signal in the target sampling signal can be easily distinguished, on the other hand, interference of the alternating current signal to the detection signal can be reduced, and a signal-to-noise ratio of the detection signal is increased accordingly.

In some possible implementation manners, a frequency of the alternating current signal is greater than a frequency of the detection signal.

The frequency of the alternating current signal is constructed to be greater than (or much greater than) the frequency of the detection signal. On one hand, the first sampling signal and the second sampling signal in the target sampling signal can be more easily distinguished, on the other hand, interference of the alternating current signal to the detection signal can be reduced, and a signal-to-noise ratio of the detection signal is increased accordingly.

In some possible implementation manners, the sampling circuit samples the differential signal at a target phase and a target frequency, and the second sampling signal is affected by at least one of the following factors: a frequency of the alternating current signal, the target frequency and the target phase.

A reasonable second sampling signal can be designed or constructed based on the factors (the frequency of the alternating current signal, the target frequency and the target phase) that affect the second sampling signal, to achieve the desired detection accuracy or accuracy rate.

In some possible implementation manners, the target phase is not equal to an integer multiple of 180 degrees.

The target phase is constructed to be not equal to an integer multiple of 180 degrees, which could reduce the difficulty in distinguishing the first sampling signal from the second sampling signal, and could improve a detection effect of the contact state detection apparatus accordingly.

In some possible implementation manners, the target phase is equal to an integer multiple of 90 degrees.

The target phase is constructed to be equal to an integer multiple of 90 degrees, which could minimize the difficulty in distinguishing the first sampling signal from the second sampling signal, and could effectively improve a detection effect of the contact state detection apparatus accordingly.

In some possible implementation manners, the target frequency is greater than or equal to the frequency of the alternating current signal, or a difference between the target frequency and the frequency of the alternating current signal is greater than a frequency of the detection signal.

By designing sampling parameters of the sampling circuit, it can be ensured that the target sampling signal includes the first sampling signal and the second sampling signal, which could improve a sampling effect of the sampling circuit and improve a detection effect of the contact state detection apparatus accordingly.

In some possible implementation manners, the target frequency is an integer multiple or a fractional multiple of the frequency of the alternating current signal.

The target frequency is constructed to be an integer multiple or a fractional multiple of the frequency of the alternating current signal, which could effectively reduce power consumption in comparison to sampling of the alternating current signal at a sampling rate that is 4 times or even higher than the frequency of the alternating current signal, and could increase the battery life of the wearable device accordingly.

In some possible implementation manners, the target frequency is greater than or equal to twice the frequency of the alternating current signal.

The target frequency is constructed to be greater than or equal to twice the frequency of the alternating current signal, which could cause the sampling of the sampling circuit to satisfy the Nyquist sampling law to ensure the sampling effect.

In some possible implementation manners, the contact state detection apparatus further includes:
a first power source;
where the first power source is respectively connected to the first electrode and the second electrode to output the alternating current signal to the first electrode and the second electrode, respectively.

In some possible implementation manners, the contact state detection apparatus further includes:
a third electrode, the third electrode being configured to receive the alternating current signal, and signals with equal frequencies, equal amplitudes and equal phases being provided between the first electrode and the third electrode, as well as between the second electrode and the third electrode.

By providing a third electrode, it is equivalent to providing a reference electrode for the first electrode and the second electrode, which could simplify the subsequent signal processing procedure for the differential signal to simplify the contact state detection apparatus and reduce the manufacturing costs.

In some possible implementation manners, the contact state detection apparatus further includes:
a second power source and a third power source;
where the second power source is respectively connected to the first electrode and the third electrode, and the third power source is respectively connected to the second electrode and the third electrode, so that signals with equal frequencies, equal frequencies amplitudes and equal frequencies phases are provided between the first electrode and the third electrode, as well as between the second electrode and the third electrode.

In some possible implementation manners, the contact state detection apparatus further includes:
an analog front end, the first electrode and the second electrode being respectively connected to the sampling circuit through the analog front end, the analog front end being configured to receive the differential signal and convert the differential signal into a digital signal, and the sampling circuit being configured to sample the digital signal and generate the target sampling signal.

In some possible implementation manners, the analog front end includes at least one of a filter, a differential amplifier and an analog-to-digital converter.

In some possible implementation manners, the contact state detection apparatus further includes:
a digital processing circuit, the digital processing circuit being connected to the sampling circuit, and the digital processing circuit being configured to receive the target sampling signal and generate the first sampling signal and the second sampling signal based on the target sampling signal.

In some possible implementation manners, the digital processing circuit is connected to a main controller of a wearable device, and the main controller is configured to control an operation of each module in the wearable device; where the main controller is further configured to receive the second sampling signal and determine whether to output a warning signal based on the second sampling signal, or the main controller is configured to receive a warning signal transmitted by the digital processing circuit, and the warning signal is used to indicate that the first electrode or the second electrode and the detected object are in a falling off state.

In a second aspect, an electronic device is provided, including:
the contact state detection apparatus in the first aspect or any one of the possible implementation manners of the first aspect.

### BRIEF DESCRIPTION OF DRAWINGS

FIG. 1 is a schematic structural diagram of a contact state detection apparatus according to an embodiment of the present application.
FIG. 2 is a schematic structural diagram of a positional relationship between either a first electrode or a second electrode in a contact state detection apparatus and a detected object according to an embodiment of the present application.
FIG. 3 is a schematic structural diagram of a contact state detection apparatus including a third electrode according to an embodiment of the present application.
FIG. 4 and FIG. 5 are both schematic diagrams of equivalent circuits formed by electrodes in a contact state detection apparatus and a detected object according to embodiments of the present application.
FIG. 6 is a schematic structural diagram of transmission signals between various devices in the contact state detection apparatus shown in FIG. 1.

### DESCRIPTION OF EMBODIMENTS

Technical solutions in embodiments of the present application will be described hereinafter with reference to accompanying drawings.

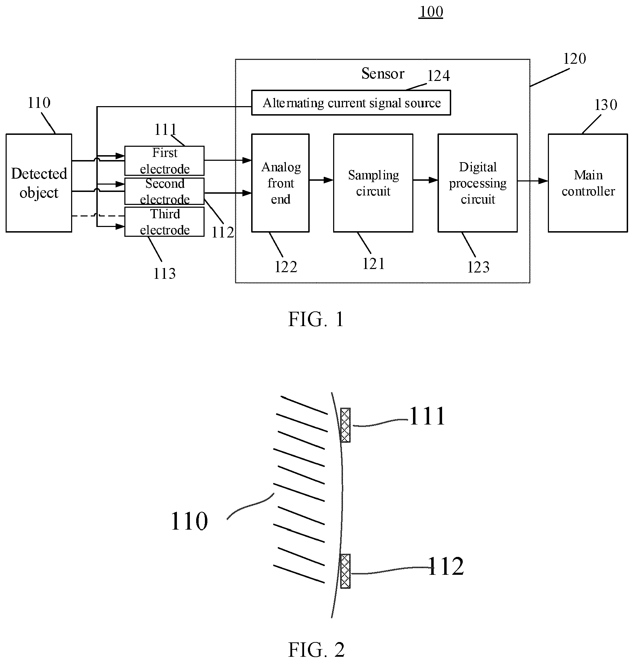

FIG. 1 is a schematic structural diagram of a contact state detection apparatus 100 according to an embodiment of the present application. It should be understood that the contact state detection apparatus 100 may be applicable to a wearable device, the wearable device includes but is not limited to a health bracelet/watch, a heart rate bracelet/watch, a Bluetooth headset, a wired headset, a handheld device, and so on. It should also be understood that the contact state detection apparatus 100 may be applicable to any electronic device that needs to detect a contact state between an electrode and a detected object. For example, the electronic device may be a medical electrocardio detection device, and the contact state detection apparatus 100 may be configured to detect a contact state between an electrode of the medical electrocardio detection device and the detected object (for example, a human body) to determine whether the electrode falls off, that is, the contact state detecting apparatus 100 may be an electrode contact state detection apparatus or an electrode fall-off detection apparatus.

As shown in FIG. 1, the contact state detection apparatus 100 may include a first electrode 111 and a second electrode 112, both of the first electrode 111 and the second electrode 112 are configured to receive an alternating current signal and a first signal to output a differential signal, the differential signal includes the first signal and a second signal, the first signal is a detection signal of physiological information of a detected object 110, and the second signal is a signal formed after the alternating current signal is modulated by the first electrode 111 and the second electrode 112. The contact state detection apparatus 100 further includes a sampling circuit 121, the sampling circuit 121 is connected to the first electrode 111 and the second electrode 112, the sampling circuit 121 is configured to sample the differential signal to obtain a target sampling signal, and the target sampling signal includes a first sampling signal corresponding to a first signal and a second sampling signal corresponding to the second signal.

The first sampling signal is used to indicate a physiological signal of the detected object 110, and the second sampling signal is used to detect a contact state of the first electrode 111 with the detected object 110 and/or a contact state of the second electrode 112 with the detected object 110.

The sampling circuit 121 may perform relevant sampling (that is, sampling where an ADC sampling frequency and an ADC sampling phase satisfy a predetermined relationship) of a signal (for example, the differential signal) through an analog-to-digital converter (ADC), sampling data (that is, the target sampling signal) of the analog-to-digital converter may be used for calculation and analysis to extract current impedance information between the first electrode 111 and/or the second electrode 112 and the detected object 110, so as to determine a contact state between the first electrode 111 and/or the second electrode 112 and the detected object 110.

In other words, the sampling circuit 121 may include an analog-to-digital converter, and the analog-to-digital converter is configured to sample the differential signal to obtain a target sampling signal.

Since a voltage difference between the first electrode and the second electrode changes when the first electrode or second electrode is in contact with the detected object (the first signal is present), differential signals output by the first electrode 111 and the second electrode 112 change accordingly. Equivalently, in a case that the first electrode 111 and/or the second electrode 112 is in contact with the detected object 110, a modulating signal formed after the alternating current signal is modulated by the first electrode 111 and the second electrode 112 changes with respect to a case that the first electrode 111 and/or the second electrode 112 is not in contact with the detected object 110. In this case, detection of physiological parameter information of the detected object 110 could be implemented through the first sampling signal in the target sampling signal. On this basis, whether the first electrode 111 or the second electrode 112 and the detected object 110 are in a contact state could be presented through the second sampling signal, to implement electrode contact state detection as well as physiological information detection at the same time.

For example, by comparing an amplitude (or an amount of reduction of an amplitude) of the second signal with a preset threshold, the contact state between the first electrode 111 and/or the second electrode 112 and the detected object 110 can be determined. Optionally, the preset threshold may be an amplitude of the second signal in a non-contact state between the first electrode 111 and/or the second electrode 112 and the detected object 110. Optionally, when the amplitude of the second signal is greater than or equal to a predetermined amplitude, the first electrode 111 or the second electrode 112 and the detected object are in a non-contact state. For example, when a positive amplitude of the second signal is greater than or equal to a first threshold, the first electrode 111 and the detected object 110 are in a non-contact state, and when an absolute value of a negative amplitude of the second signal is greater than or equal to a second threshold, the second electrode 112 and the detected object 110 are in a non-contact state. The first threshold and the second threshold may be equal or unequal. Optionally, the first threshold and the second threshold may be thresholds for electrode contact state detection by the wearable device. Optionally, the first threshold and the second threshold may be greater than a threshold for lead contact state detection by medical specialized equipment, so as to avoid false detection due to high contact impedance.

In addition, by driving the first electrode 111 and the second electrode 112 by the alternating current signal to detect the physiological information of the detected object 110, the alternating current signal in the target sampling signal that is modulated by the first electrode 111 and the second electrode 112 can be multiplexed into a signal used to indicate a contact state of the first electrode 111 and/or the second electrode 112 with the detected object 110, such as a signal indicating whether the first electrode 111 and/or the second electrode 112 falls off, while the physiological information detection is implemented, which could reduce devices in the contact state detection apparatus 110, simplify the apparatus and reduce the manufacturing costs accordingly.

In addition, sampling parameters of the sampling circuit 121 may also be designed to improve a sampling effect of the sampling circuit 121 on the differential signal.

In some embodiments of the present application, the sampling circuit 121 may sample the differential signal at a target phase and a target frequency, and the second sampling signal is affected by at least one of the following factors: a frequency of the alternating current signal, the target frequency and the target phase.

For example, a signal type of the second sampling signal is affected by at least one of the following factors: a frequency of the alternating current signal, the target frequency and the target phase. Optionally, a type of the second sampling signal includes but is not limited to a direct current signal type and an alternating current signal type, and the alternating current signal type includes but is not limited to alternating current signals at different frequencies. For example, if the target phase is an integer multiple of 90 degrees and the target frequency is equal to the frequency of the alternating current signal, the first sampling signal is a direct current signal; and if the target phase is an integer multiple of 90 degrees and the target frequency is not equal to the frequency of the alternating current signal, the first sampling signal is an alternating current signal.

A reasonable second sampling signal can be designed or constructed based on the factors (the frequency of the alternating current signal, the target frequency and the target phase) that affect the second sampling signal, to achieve the desired detection accuracy or accuracy rate.

In other words, based on circuit structures among the first electrode 111, the second electrode 112 and the sampling circuit 121 in the present application, the alternating current signals for driving the first electrode 111 and the second electrode 112 and the sampling parameters of the sampling circuit 121 may be designed to obtain the desired second sampling signals.

For example, "the target phase is not equal to an integer multiple of 180 degrees, which thus could reduce the difficulty in distinguishing the first sampling signal from the second sampling signal, and could improve a detection effect of the contact state detection apparatus 100 accordingly. For another example, the target phase is equal to an integer multiple of 90 degrees, which thus could minimize the difficulty in distinguishing the first sampling signal from the second sampling signal, and could effectively improve a detection effect of the contact state detection apparatus accordingly. Optionally, the target phase may be a phase at which the sampling circuit 121 is configured to sample the differential signal when a phase of the alternating current signal is zero phase.

For another example, the target frequency is greater than or equal to the frequency of the alternating current signal, or a difference between the target frequency and the frequency of the alternating current signal is greater than a frequency of the detection signal. Accordingly, it can be ensured that the target sampling signal includes the first sampling signal and the second sampling signal, which could improve a sampling effect of the sampling circuit 121 and improve a detection effect of the contact state detection apparatus 100 accordingly. Optionally, the target frequency is an integer multiple or a fractional multiple of the frequency of the alternating current signal. For example, the target frequency is greater than or equal to twice the frequency of the alternating current signal. The target frequency is constructed to be greater than or equal to twice the frequency of the alternating current signal, which could cause the sampling of the sampling circuit 121 to satisfy the Nyquist sampling law to ensure the sampling effect.

In addition, the alternating current signal may also be designed to increase a signal-to-noise ratio of the detection signal.

For example, the alternating current signal is a high-frequency signal. Since the detection signal is a low-frequency signal, the alternating current signal is constructed as a high-frequency signal. On one hand, the first sampling signal and the second sampling signal in the target sampling signal can be easily distinguished, on the other hand, interference of the alternating current signal to the detection signal can be reduced, and a signal-to-noise ratio of the detection signal is increased accordingly.

For another example, the frequency of the alternating current signal is greater than the frequency of the detection signal. By constructing the frequency of the alternating current signal to be greater than (or much greater than) the frequency of the detection signal, on one hand, the first sampling signal and the second sampling signal in the target sampling signal can be more easily distinguished, on the other hand, interference of the alternating current signal to the detection signal can be reduced, and a signal-to-noise ratio of the detection signal is increased accordingly.

As shown in FIG. 1, in some embodiments of the present application, the contact state detection apparatus 100 may further include a third electrode 113, the third electrode 113 is configured to receive the alternating current signal, and signals with equal frequencies, equal amplitudes and equal phases are provided between the first electrode 111 and the third electrode 113, as well as between the second electrode 112 and the third electrode 113.

By providing a third electrode 113, it is equivalent to providing a reference electrode for the first electrode 111 and the second electrode 112, which could simplify the subsequent signal processing procedure for the differential signal to simplify the contact state detection apparatus 110 and reduce the manufacturing costs.

As shown in FIG. 1, in some embodiments of the present application, the contact state detection apparatus 100 may further include an alternating current signal source 124, the alternating current signal source 124 is connected to the first electrode 111 and the second electrode 112, and the alternating current signal source 124 is configured to generate the alternating current signal. Optionally, the alternating current signal source 124 may be a current source or a voltage source. Optionally, the alternating current signal may be a sine wave or a square wave.

As shown in FIG. 1, in some embodiments of the present application, the contact state detection apparatus 100 further include an analog front end 122, the first electrode 111 and the second electrode 112 are respectively connected to the sampling circuit 121 through the analog front end 122, the analog front end 122 is configured to receive the differential signal and convert the differential signal into a digital signal, and the sampling circuit 121 is configured to sample the digital signal and generate the target sampling signal.

For example, the analog front end 122 includes at least one of a filter, a differential amplifier and an analog-to-digital converter. Specifically, the filter filters out a noise signal in the received signal, the differential amplifier performs differential amplification on the received differential signal, and the amplified signal is converted into a digital signal by the analog-to-digital converter, so that the sampling circuit 121 can sample the digital signal and output the target sampling signal.

As shown in FIG. 1, in some embodiments of the present application, the contact state detection apparatus 100 further includes a digital processing circuit 123, the digital processing circuit 123 is connected to the sampling circuit 121, and the digital processing circuit 121 is configured to receive the target sampling signal and generate the first sampling signal and the second sampling signal based on the target sampling signal.

As shown in FIG. 1, in an actual product, the alternating current signal source 124, the analog front end 122, the sampling circuit 121 and the digital processing circuit 123 may serve as devices in a sensor 120 to increase integration of the contact state detection apparatus, reduce the occupied volume of the contact state detection apparatus accordingly, and improve its applicability in the wearable device.

As shown in FIG. 1, in some embodiments of the present application, the digital processing circuit 123 is connected to a main controller 130 of the wearable device, and the main controller 130 is configured to control an operation of each module in the wearable device; where the main controller 130 is further configured to receive the second sampling signal and determine whether to output a warning signal based on the second sampling signal, or the main controller 130 is configured to receive the warning signal transmitted by the digital processing circuit, and the warning signal is used to indicate that the first electrode 111 or the second electrode 112 and the detected object 110 are in a falling off state.

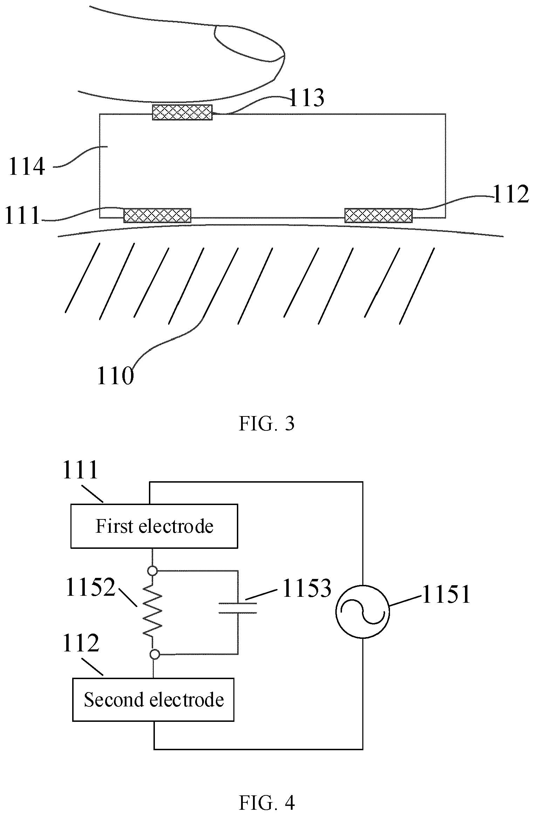

FIG. 2 and FIG 3 are both schematic structural diagrams of positional relationships between electrodes and a detected object according to embodiments of the present application.

As shown in FIG. 2, if the contact state detection apparatus 100 only includes the first electrode 111 and the second electrode 112, both the first electrode 111 and the second electrode 112 are in contact with the detected object 110.

As shown in FIG. 3, if the contact state detection apparatus 100 further includes a third electrode 113, both the first electrode 111 and the second electrode 112 are in contact with the detected object 110. The third electrode 113 may be in contact with the detected object 110, or may not be in contact with the detected object 110.

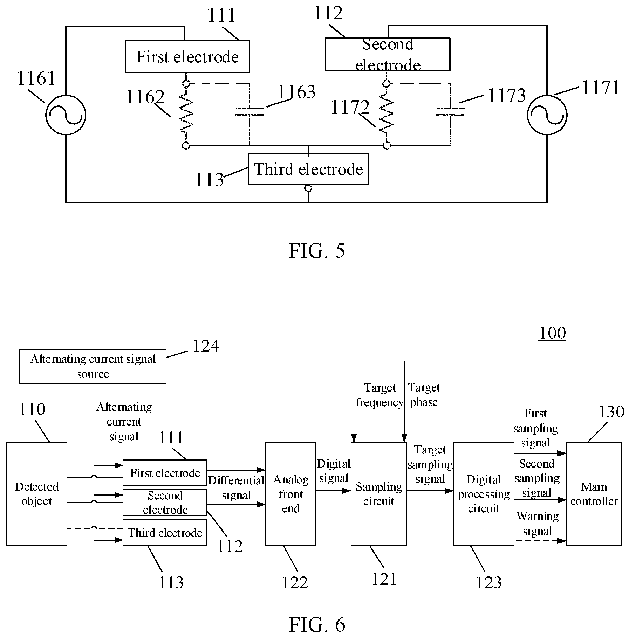

FIG. 4 and FIG. 5 are both schematic diagrams of equivalent circuits formed by electrodes in a contact state detection apparatus and a detected object according to embodiments of the present application.

As shown in FIG. 4, in some embodiments of the present application, the contact state detection apparatus 100 further includes a first power source1151, the first power source 1151 is respectively connected to the first electrode 111 and the second electrode 112 to output the alternating current signal to the first electrode 111 and the second electrode 112, respectively. A first resistor 1152 and a first capacitor1153 are formed between the first electrode 111 and the detected object.

In other words, an equivalent circuit formed by the first electrode 111, the second electrode 112 and the first power source 1151 can be described as: one end of the first power source 1151 is connected to the first electrode 111, the other end of the first power source 1151 is connected to the second electrode 112, the first electrode 111 is connected to the second electrode 112 through the first resistor 1152, and the first capacitor 1153 is connected in parallel to the first resistor 1152.

As shown in FIG. 5, in some other embodiments of the present application, the contact state detection apparatus 100 further includes a second power source 1161 and a third power source 1171, the second power source 1161 is respectively connected to the first electrode 111 and the third electrode 113, and the third power source 1171 is respectively connected to the second electrode 112 and the third electrode 113, so that signals with equal frequencies, equal amplitudes and equal phases are provided between the first electrode 111 and the third electrode 113, as well as between the second electrode 112 and the third electrode 113.

In other words, an equivalent circuit formed by the first electrode 111, the second electrode 112, the third electrode 113 and the first power source 1151 can be described as: one end of the third electrode 113 is connected to one end of the first electrode 111 through the second power source 1161 and connected to one end of the second electrode 112 through the third power source 1171, the other end of the third electrode 113 is connected to the other end of the first electrode 111 through the second resistor 1162 and connected to the other end of the second electrode 112 through the third resistor 1172, the second capacitor 1163 is connected in parallel to the second resistor 1162, and the third capacitor 1173 is connected in parallel to the third resistor 1172.

FIG. 6 is a schematic structural diagram of transmission signals between various devices in the contact state detection apparatus shown in FIG. 1.

As shown in FIG. 6, in the contact state detection apparatus 100, both of the first electrode 111 and the second electrode 112 output a differential signal to the analog front end 122, so that the analog front end 122 converts the differential signal into a digital signal, the sampling circuit 121 samples the digital signal with a signal having a target frequency and a target phase to output the target sampling signal, and the digital processing circuit 123 generates the first sampling signal and the second sampling signal based on the target sampling signal, and outputs the first sampling signal and the second sampling signal to the main controller 130. Optionally, the digital processing circuit 123 may further generate the foregoing warning signal based on the second sampling signal, and output the warning signal to the main controller 130.

In addition, the present application provides a wearable device, and the wearable device may include the foregoing contact state detection apparatus 100.

Those of ordinary skill in the art may be aware that, units and algorithm steps of the examples described in the embodiments disclosed herein may be implemented by electronic hardware, or a combination of computer software and computer software. Whether these functions are executed in hardware or software mode depends on the specific applications and design constraint conditions of the technical solution. Those skilled may implement the described functions by using different methods for each specific application, but this implementation should not be considered to be beyond the scope of the present application.

In addition, functional units in the embodiments of the present application may be integrated into one processing unit, or each of the units may exist alone physically, or two or more units are integrated into one unit.

When the functions are implemented in the form of a software functional unit and sold or used as an independent product, the functions may be stored in a computer-readable storage medium. Based on such understanding, the technical solutions of the present application substantially, or the part of the present application making contribution to the prior art, or a part of the technical solution may be embodied in the form of a software product, and the computer software product is stored in a storage medium, which includes multiple instructions enabling computer equipment (which may be a personal computer, a server, network equipment or the like) to execute all of or part of the steps in the methods of the embodiments of the present application. The foregoing storage medium includes: various media capable of storing program codes, such as a U disk, a mobile hard disk, a read-only memory (ROM, Read-Only Memory), a random access memory (RAM, Random Access Memory), a disk, an optical disk or the like.

Described above are the specific implementation manners of the present application only, but the protection scope of the present application is not limited thereto, those skilled who are familiar with the art could readily think of variations or substitutions within the technical scope disclosed by the present application, and these variations or substitutions shall fall within the protection scope of the present application. Therefore, the protection scope of the present application shall be subject to the protection scope of the claims.

## Claims

1. A contact state detection apparatus (100), wherein the contact state detection apparatus (100) comprises:
a first electrode (111) and a second electrode (112), the first electrode (111) and the second electrode (112) being configured to receive an alternating current signal and a first signal to output a differential signal, the differential signal comprising the first signal and a second signal, the first signal being a detection signal of physiological information of a detected object (110), and the second signal being a signal formed after the alternating current signal is modulated by the first electrode (111) and the second electrode (112); and
a sampling circuit (121), the sampling circuit (121) being connected to the first electrode (111) and the second electrode (112), the sampling circuit (121) being configured to sample the differential signal to obtain a target sampling signal, and the target sampling signal comprising a first sampling signal corresponding to a first signal and a second sampling signal corresponding to the second signal;
wherein the first sampling signal is used to indicate a physiological signal of the detected object (110), and the second sampling signal is used to detect a contact state of the first electrode (111) with the detected object (110) and/or a contact state of the second electrode (112) with the detected object (110).

2. The contact state detection apparatus (100) according to claim 1, wherein the alternating current signal is a high-frequency signal.

3. The contact state detection apparatus (100) according to claim 1 or 2, wherein a frequency of the alternating current signal is greater than a frequency of the detection signal.

4. The contact state detection apparatus (100) according to any one of claims 1 to 3, wherein the sampling circuit (121) samples the differential signal at a target phase and a target frequency, and the second sampling signal is affected by at least one of the following factors: a frequency of the alternating current signal, the target frequency and the target phase.

5. The contact state detection apparatus (100) according to claim 4, wherein the target phase is not equal to an integer multiple of 180 degrees.

6. The contact state detection apparatus (100) according to claim 4, wherein the target phase is equal to an integer multiple of 90 degrees.

7. The contact state detection apparatus (100) according to claim 4, wherein the target frequency is greater than or equal to the frequency of the alternating current signal, or a difference between the target frequency and the frequency of the alternating current signal is greater than a frequency of the detection signal.

8. The contact state detection apparatus (100) according to claim 7, wherein the target frequency is an integer multiple or a fractional multiple of the frequency of the alternating current signal.

9. The contact state detection apparatus (100) according to claim 7, wherein the target frequency is greater than or equal to twice the frequency of the alternating current signal.

10. The contact state detection apparatus (100) according to any one of claims 1 to 9, wherein the contact state detection apparatus further comprises:
a first power source (1151);
wherein the first power source (1151) is respectively connected to the first electrode (111) and the second electrode (112) to output the alternating current signal to the first electrode (111) and the second electrode (112), respectively.

11. The contact state detection apparatus (100) according to any one of claims 1 to 9, wherein the contact state detection apparatus (100) further comprises:
a third electrode (113), the third electrode (113) being configured to receive the alternating current signal, and signals with equal frequencies, equal amplitudes and equal phases being provided between the first electrode (111) and the third electrode (113), as well as between the second electrode (112) and the third electrode (113).

12. The contact state detection apparatus (100) according to claim 11, wherein the contact state detection apparatus (100) further comprises:
a second power source (1161) and a third power source (1171);
wherein the second power source (1161) is respectively connected to the first electrode (111) and the third electrode (113), and the third power source (1171) is respectively connected to the second electrode (112) and the third electrode (113), so that signals with equal frequencies, equal amplitudes and equal phases are provided between the first electrode (111) and the third electrode (113), as well as between the second electrode (112) and the third electrode (113).

13. The contact state detection apparatus (100) according to any one of claims 1 to 12, wherein the contact state detection apparatus (100) further comprises:
an analog front end (122), the first electrode (111) and the second electrode (112) being respectively connected to the sampling circuit (121) through the analog front end (122), the analog front end (122) being configured to receive the differential signal and convert the differential signal into a digital signal, and the sampling circuit (121) being configured to sample the digital signal and generate the target sampling signal.

14. The contact state detection apparatus (100) according to any one of claims 1 to 13, wherein the contact state detection apparatus further comprises:
a digital processing circuit (123), the digital processing circuit (123) being connected to the sampling circuit (121), and the digital processing circuit (123) being configured to receive the target sampling signal and generate the first sampling signal and the second sampling signal based on the target sampling signal.

15. A wearable device, wherein the wearable device comprises:
the contact state detection apparatus (100) according to any one of claims 1 to 14.

## Patentansprüche

1. Kontaktzustand-Erfassungsvorrichtung (100), wobei die Kontaktzustand-Erfassungsvorrichtung (100) umfasst:
eine erste Elektrode (111) und eine zweite Elektrode (112), wobei die erste Elektrode (111) und die zweite Elektrode (112) dazu konfiguriert sind, ein Wechselstromsignal und ein erstes Signal zu empfangen, um ein Differenzsignal auszugeben, wobei das Differenzsignal das erste Signal und ein zweites Signal umfasst, wobei das erste Signal ein Erfassungssignal von physiologischen Informationen eines erfassten Objekts (110) ist und das zweite Signal ein Signal ist, das gebildet wird, nachdem das Wechselstromsignal durch die erste Elektrode (111) und die zweite Elektrode (112) moduliert worden ist; und
eine Abtastschaltung (121), wobei die Abtastschaltung (121) mit der ersten Elektrode (111) und der zweiten Elektrode (112) verbunden ist, wobei die Abtastschaltung (121) dazu konfiguriert ist, das Differenzsignal abzutasten, um ein Zielabtastsignal zu erhalten, und wobei das Zielabtastsignal ein erstes Abtastsignal, das einem ersten Signal entspricht, und ein zweites Abtastsignal, das dem zweiten Signal entspricht, umfasst;
wobei das erste Abtastsignal verwendet wird, um ein physiologisches Signal des erfassten Objekts (110) anzuzeigen, und das zweite Abtastsignal verwendet wird, um einen Kontaktzustand der ersten Elektrode (111) mit dem erfassten Objekt (110) und/oder einen Kontaktzustand der zweiten Elektrode (112) mit dem erfassten Objekt (110) zu erfassen.

2. Kontaktzustand-Erfassungsvorrichtung (100) nach Anspruch 1, wobei das Wechselstromsignal ein Hochfrequenzsignal ist.

3. Kontaktzustand-Erfassungsvorrichtung (100) nach Anspruch 1 oder 2, wobei eine Frequenz des Wechselstromsignals größer ist als eine Frequenz des Erfassungssignals.

4. Kontaktzustand-Erfassungsvorrichtung (100) nach einem der Ansprüche 1 bis 3, wobei die Abtastschaltung (121) das Differenzsignal mit einer Zielphase und einer Zielfrequenz abtastet, und das zweite Abtastsignal von wenigstens einem der folgenden Faktoren beeinflusst wird: eine Frequenz des Wechselstromsignals, die Zielfrequenz und die Zielphase.

5. Kontaktzustand-Erfassungsvorrichtung (100) nach Anspruch 4, wobei die Zielphase nicht gleich einem ganzzahligen Vielfachen von 180 Grad ist.

6. Kontaktzustand-Erfassungsvorrichtung (100) nach Anspruch 4, wobei die Zielphase gleich einem ganzzahligen Vielfachen von 90 Grad ist.

7. Kontaktzustand-Erfassungsvorrichtung (100) nach Anspruch 4, wobei die Zielfrequenz größer oder gleich der Frequenz des Wechselstromsignals ist, oder eine Differenz zwischen der Zielfrequenz und der Frequenz des Wechselstromsignals größer ist als eine Frequenz des Erfassungssignals.

8. Kontaktzustand-Erfassungsvorrichtung (100) nach Anspruch 7, wobei die Zielfrequenz ein ganzzahliges Vielfaches oder ein gebrochenes Vielfaches der Frequenz des Wechselstromsignals ist.

9. Kontaktzustand-Erfassungsvorrichtung (100) nach Anspruch 7, wobei die Zielfrequenz größer oder gleich dem Doppelten der Frequenz des Wechselstromsignals ist.

10. Kontaktzustand-Erfassungsvorrichtung (100) nach einem der Ansprüche 1 bis 9, wobei die Kontaktzustand-Erfassungsvorrichtung ferner umfasst:
eine erste Energiequelle (1151);
wobei die erste Energiequelle (1151) jeweils mit der ersten Elektrode (111) und der zweiten Elektrode (112) verbunden ist, um das Wechselstromsignal an die erste Elektrode (111) bzw. die zweite Elektrode (112) auszugeben.

11. Kontaktzustand-Erfassungsvorrichtung (100) nach einem der Ansprüche 1 bis 9, wobei die Kontaktzustand-Erfassungsvorrichtung (100) ferner umfasst:
eine dritte Elektrode (113), wobei die dritte Elektrode (113) dazu konfiguriert ist, das Wechselstromsignal zu empfangen, wobei Signale mit gleichen Frequenzen, gleichen Amplituden und gleichen Phasen zwischen der ersten Elektrode (111) und der dritten Elektrode (113), sowie zwischen der zweiten Elektrode (112) und der dritten Elektrode (113) bereitgestellt werden.

12. Kontaktzustand-Erfassungsvorrichtung (100) nach Anspruch 11, wobei die Kontaktzustand-Erfassungsvorrichtung (100) ferner umfasst:
eine zweite Energiequelle (1161) und eine dritte Energiequelle (1171);
wobei die zweite Energiequelle (1161) jeweils mit der ersten Elektrode (111) und der dritten Elektrode (113) verbunden ist und die dritte Energiequelle (1171) jeweils mit der zweiten Elektrode (112) und der dritten Elektrode (113) verbunden ist, so dass Signale mit gleichen Frequenzen, gleichen Amplituden und gleichen Phasen zwischen der ersten Elektrode (111) und der dritten Elektrode (113), sowie zwischen der zweiten Elektrode (112) und der dritten Elektrode (113) bereitgestellt werden.

13. Kontaktzustand-Erfassungsvorrichtung (100) nach einem der Ansprüche 1 bis 12, wobei die Kontaktzustand-Erfassungsvorrichtung (100) ferner umfasst:
ein analoges Front-End (122), wobei die erste Elektrode (111) und die zweite Elektrode (112) jeweils über das analoge Front-End (122) mit der Abtastschaltung (121) verbunden sind, wobei das analoge Front-End (122) dazu konfiguriert ist, das Differenzsignal zu empfangen und das Differenzsignal in ein digitales Signal zu konvertieren, und wobei die Abtastschaltung (121) dazu konfiguriert ist, das digitale Signal abzutasten und das Zielabtastsignal zu erzeugen.

14. Kontaktzustand-Erfassungsvorrichtung (100) nach einem der Ansprüche 1 bis 13, wobei die Kontaktzustand-Erfassungsvorrichtung ferner umfasst:
eine digitale Verarbeitungsschaltung (123), wobei die digitale Verarbeitungsschaltung (123) mit der Abtastschaltung (121) verbunden ist und die digitale Verarbeitungsschaltung (123) dazu konfiguriert ist, das Zielabtastsignal zu empfangen und das erste Abtastsignal und das zweite Abtastsignal basierend auf dem Zielabtastsignal zu erzeugen.

15. Tragbare Vorrichtung, wobei die tragbare Vorrichtung umfasst:
die Kontaktzustand-Erfassungsvorrichtung (100) nach einem der Ansprüche 1 bis 14.

## Revendications

1. Appareil de détection d'état de contact (100), dans lequel l'appareil de détection d'état de contact (100) comprend :
une première électrode (111) et une deuxième électrode (112), la première électrode (111) et la deuxième électrode (112) étant configurées pour recevoir un signal de courant alternatif et un premier signal pour délivrer en sortie un signal différentiel, le signal différentiel comprenant le premier signal et un deuxième signal, le premier signal étant un signal de détection d'informations physiologiques d'un objet détecté (110), et le deuxième signal étant un signal formé après la modulation du signal de courant alternatif par la première électrode (111) et la deuxième électrode (112) ; et
un circuit d'échantillonnage (121), le circuit d'échantillonnage (121) étant relié à la première électrode (111) et à la deuxième électrode (112), le circuit d'échantillonnage (121) étant configuré pour échantillonner le signal différentiel pour obtenir un signal d'échantillonnage cible et le signal d'échantillonnage cible comprenant un premier signal d'échantillonnage correspondant à un premier signal et un deuxième signal d'échantillonnage correspondant au deuxième signal ;
dans lequel le premier signal d'échantillonnage est utilisé pour indiquer un signal physiologique de l'objet détecté (110), et le deuxième signal d'échantillonnage est utilisé pour détecter un état de contact de la première électrode (111) avec l'objet détecté (110) et/ou un état de contact de la deuxième électrode (112) avec l'objet détecté (110).

2. Appareil de détection d'état de contact (100) selon la revendication 1, dans lequel le signal de courant alternatif est un signal haute fréquence.

3. Appareil de détection d'état de contact (100) selon la revendication 1 ou 2, dans lequel une fréquence du signal de courant alternatif est supérieure à une fréquence du signal de détection.

4. Appareil de détection d'état de contact (100) selon l'une quelconque des revendications 1 à 3, dans lequel le circuit d'échantillonnage (121) échantillonne le signal différentiel à une phase cible et une fréquence cible, et le deuxième signal d'échantillonnage est affecté par au moins un des facteurs suivants : une fréquence du signal de courant alternatif, la fréquence cible et la phase cible.

5. Appareil de détection d'état de contact (100) selon la revendication 4, dans lequel la phase cible n'est pas égale à un nombre entier multiple de 180 degrés.

6. Appareil de détection d'état de contact (100) selon la revendication 4, dans lequel la phase cible est égale à un nombre entier multiple de 90 degrés.

7. Appareil de détection d'état de contact (100) selon la revendication 4, dans lequel la fréquence cible est supérieure ou égale à la fréquence du signal de courant alternatif, ou une différence entre la fréquence cible et la fréquence du signal de courant alternatif est supérieure à une fréquence du signal de détection.

8. Appareil de détection d'état de contact (100) selon la revendication 7, dans lequel la fréquence cible est un nombre entier multiple ou un multiple fractionné de la fréquence du signal de courant alternatif.

9. Appareil de détection d'état de contact (100) selon la revendication 7, dans lequel la fréquence cible est supérieure ou égale à deux fois la fréquence du signal de courant alternatif.

10. Appareil de détection d'état de contact (100) selon l'une quelconque des revendications 1 à 9, dans lequel l'appareil de détection d'état de contact comprend en outre :
une première source d'énergie (1151) ;
dans lequel la première source d'énergie (1151) est reliée respectivement à la première électrode (111) et à la deuxième électrode (112) pour délivrer en sortie le signal de courant alternatif à la première électrode (111) et à la deuxième électrode (112) respectivement.

11. Appareil de détection d'état de contact (100) selon l'une quelconque des revendications 1 à 9, dans lequel l'appareil de détection d'état de contact (100) comprend en outre :
une troisième électrode (113), la troisième électrode (113) étant configurée pour recevoir le signal de courant alternatif, et des signaux à fréquences égales, à amplitudes égales et à phases égales étant fournis entre la première électrode (111) et la troisième électrode (113) ainsi qu'entre la deuxième électrode (112) et la troisième électrode (113).

12. Appareil de détection d'état de contact (100) selon la revendication 11, dans lequel l'appareil de détection d'état de contact (100) comprend en outre :
une deuxième source d'énergie (1161) et une troisième source d'énergie (1171) ;
dans lequel la deuxième source d'énergie (1161) est reliée respectivement à la première électrode (111) et à la troisième électrode (113), et la troisième source d'énergie (1171) est reliée respectivement à la deuxième électrode (112) et à la troisième électrode (113) de telle sorte que des signaux à fréquences égales, à amplitudes égales et à phases égales sont fournis entre la première électrode (111) et la troisième électrode (113) tout comme entre la deuxième électrode (112) et la troisième électrode (113).

13. Appareil de détection d'état de contact (100) selon l'une quelconque des revendications 1 à 12, dans lequel l'appareil de détection d'état de contact (100) comprend en outre :
une entrée analogique (122), la première électrode (111) et la deuxième électrode (112) étant reliées respectivement au circuit d'échantillonnage (121) par l'intermédiaire de l'entrée analogique (122), l'entrée analogique (122) étant configurée pour recevoir le signal différentiel et pour convertir le signal différentiel en un signal numérique, et le circuit d'échantillonnage (121) étant configuré pour échantillonner le signal numérique et pour générer le signal d'échantillonnage cible.

14. Appareil de détection d'état de contact (100) selon l'une quelconque des revendications 1 à 13, dans lequel l'appareil de détection d'état de contact comprend en outre :
un circuit de traitement numérique (123), le circuit de traitement numérique (123) étant relié au circuit d'échantillonnage (121), et le circuit de traitement numérique (123) étant configuré pour recevoir le signal d'échantillonnage cible et pour générer le premier signal d'échantillonnage et le deuxième signal d'échantillonnage sur la base du signal d'échantillonnage cible.

15. Appareil vestimentaire, dans lequel l'appareil vestimentaire comprend :
l'appareil de détection d'état de contact (100) selon l'une quelconque des revendications 1 à 14.
